# EUROPEAN PATENT SPECIFICATION

(11) **EP 1 438 058 B1**
(45) Date of publication and mention of the grant of the patent: **14.01.2009**
(21) Application number: 02785193.0
(22) Date of filing: 10.10.2002
(51) Int. Cl.: A61K 36/48, A61P 35/00, A61P 39/00

(54) **AQUEOUS EXTRACT OF CERATONIA SILIQUA LEAVES AND PODS CONTAINING POLYPHENOLS WITH ANTIOXIDANT AND ANTITUMOR ACTIVITIES**
WAESSRIGER EXTRAKT AUS BLÄTTERN UND HÜLSEN VON CERATONIA SILIQUA ENTHALTEND POLYPHENOLEN MIT ANTIOXIDATIVER UND ANTITUMORALER WIRKUNG
EXTRAIT AQUEUX DE FEUILLES ET DE GOUSSES DE CERATONIA SILIQUA CONTENANT DES POLYPHENOLS AYANT DES ACTIVITES ANTITUMORALE ET ANTIOXYDANTE

(30) Priority: 17.10.2001 IT MI20012141
(43) Date of publication of application: 21.07.2004
(73) Proprietor: Baraldi, Mario, 41100 Modena (IT)
(72) Inventor: Baraldi, Mario, 41100 Modena (IT)
(74) Representative: Bianchetti, Giuseppe
(86) International application number: PCT/EP2002/011356
(87) International publication number: WO 2003/033006

(56) References cited:
- WO-A-93/19765
- US-A- 4 741 915
- US-A- 4 999 197
- CANTALEJO, M.J.: "Antioxidant and immunomodulating activities of condensed tannins from carob pods" RECENT RES. DEVEP. AGRICULTURAL & FOOD CHEM., vol. 5, 2001, pages 101-111, XP009004016
- CANTALEJO M.J.: "Effects of condensed polyphenols from carob pods on bioactivity assays" POLYPHENOL COMMUNICATIONS 98, 1998, pages 27-28, XP001246457

## Description

The present invention relates to an aqueous extract of *Ceratonia siliqua* leaves and pod containing polyphenols (gallic acid, (-)-epigallocatechin gallate, (-)-epicatechin gallate) with antioxidant activity and potential antitumor activity, useful for the preparation of pharmaceutical compositions, dietary supplements or extemporaneous preparations for the alimentary use. Said extract is characterized by high content in polyphenols as well as absence of central nervous system-stimulating substances, such as caffeine and theophylline.

### TECHNICAL BACKGROUND

According to numerous papers in literature, diet rich in fruit and vegetables apparently concurs in preventing tumors of the gastrointestinal tract [J.M. Pezzetto, Biochem. Pharmacol. 53:121-123, 1997]. Vegetables, in fact, contain a number of compounds with antioxidant and antitumor activities, inter alia flavonoids and polyphenols, which are considered able to interfere with the hepatic microsomal system, preventing it from activating the cancerogenic substances found in food and environment.

Flavonoids are a heterogeneous group of vegetable polyphenols (more than 4000) sharing a benzopyrane structure. They constitute pigments (orange, red and blue colours) of various vegetable (flowers, fruit, leaves) and are important factors for the growth, development and protection of plants. The major flavonoids with antioxidant activity present in vegetables are: (+)-catechin, (-)-epicatechin, (-)-epigallocatechin and the corresponding gallic acid esters: (-)-epicatechin gallate and (-)-epigallocatechin gallate [Blot et al. Am. J. Cancer Prevention 62:1477-82, 1992].

A number of studies reported the antitumor activity of these compounds [Cao Y. and Cao R. Nature 398: 391, 1999], in particular of (-)-epigallocatechin gallate (EGCG). EGCG takes part in:
- regulating the cellular cycle, in particular through dose-dependent inhibition of mitosis by blocking cells in the G0-G1 phase;
- inducing apoptosis (programmed cell death) in various tumour cells, but not in normal cells. This process is important for maintaining cell growth and proliferation balances. Tumour cells growth is in fact due to escape from the normal control mechanisms, which also act through regulation of apoptosis;
- inhibiting urokinase, a proteolytic enzyme which allows tumour cells to invade the adjacent cells and form metastasis;
- inhibiting angiogenesis;
- eliminating free radicals thanks to its high antioxidant power.

Among the commercially available vegetable products, green and black teas have antioxidant and antitumour activities, due to their high contents in flavonoids; however, not all individuals can tolerate them due to the presence of stimulant substances such as caffeine and theophylline. Vegetable products containing flavonoids but free from any stimulating substances, for use in the prevention and/or treatment of degenerative diseases and in particular of tumors [Fujiki H. et al. Nutrition Reviews 54: 67-70, 1996] would therefore be valuable. Cantalejo [Polyphenol Communications 98, 1998, p. 27-28] describes the antioxidative and anticarcinogenic activity of the condensed polyphenols present in extract from carob pods.

The present invention relates to an aqueous extract which has high content in polyphenols while being free from stimulating substances, obtained from carob (an evergreen tree with wide leafage, belonging to the order *Rosales,* family *Papillionacee,* sub-family *Cesalpinoidee,* genus *Ceratonia,* species *Ceratonia siliqua*).

### DETAILED DISCLOSURE OF THE INVENTION

The present invention relates to the use of a dry or aqueous extract of *Ceratonia siliqua* (carob) leaves and pod, containing polyphenols, for the preparation of pharmaceutical compositions, dietary supplements or extemporaneous preparations for the alimentary use, having antioxidant activity and potential antitumor activity. The term "polyphenols" herein preferably means gallic acid, (-)-epigallocatechin gallate and (-)-epicatechin gallate.

The present invention further relates to the process for the preparation of said extract.

Carob pod leaves or meal infused in hot distilled water for 15 min, preferably in a ratio of 1 g of leaves or meal to 50-200 ml of water, most preferably 1 g of leaves or meal to 100 ml of water. The aqueous extract is then centrifuged and filtered. A second aqueous extract is optionally obtained by further infusing the centrifugation residue. The aqueous extract or the combined aqueous extracts are evaporated to obtain a dry extract, which consists solely of water-soluble substances and can therefore be easily dissolved and is well-suited for the preparation of formulations such as sachets, syrups, capsules. This extract is free from central nervous system-stimulating substances: as a consequence, it is well tolerated and does not require costly decaffeinization or deteination processes.

According to a preferred embodiment of the invention, the extract is prepared by infusing 1 g of carob leaves or pod meal in 100 ml of hot distilled water for 15 min. The aqueous extract is then centrifuged at 13,000 rpm for 10 min and filtered through PTFE 0.22 µm filters. A second aqueous extract is obtained repeating the procedure on the centrifugation residue.

Carob leaves and pod contain fair amounts of polyphenols, mainly catechins (gallic acid, (-) epicatechin gallate, (-) epigallocatechin gallate), which have been separated and purified by chromatography (HPLC). The obtained amounts are (Table n°1 example 2) 6.28 mg/g dry leaves for the leaves and 1.36 mg/g of meal for the pod meal, respectively.

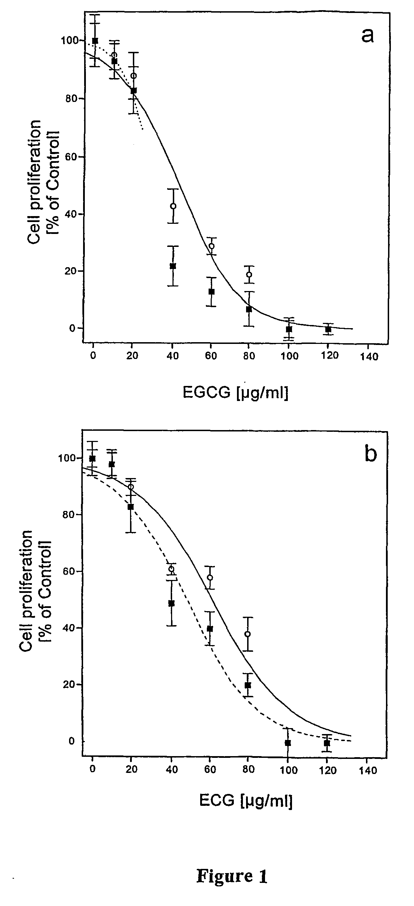

Biological tests carried out using the above mentioned catechins evidenced a marked, dose-dependent reduction in the proliferation of hepatic tumour cells cultures. Figure 1 shows the dose-dependent activities of EGCG (a) and EGC (b) on the inhibition of cell proliferation in liver tumour cells after 24 (white dots) and 48 hours (black dots) exposure. (-)-Epigallocatechin gallate (EGCG) and (-)-epicatechin gallate (ECG) inhibit the proliferation already at concentrations of 40 µg/ml, reaching the peak activity at concentrations of 80 µg/ml. Furthermore, said reduction turned out to be higher for 48 hours treatments compared with 24 hours treatments.

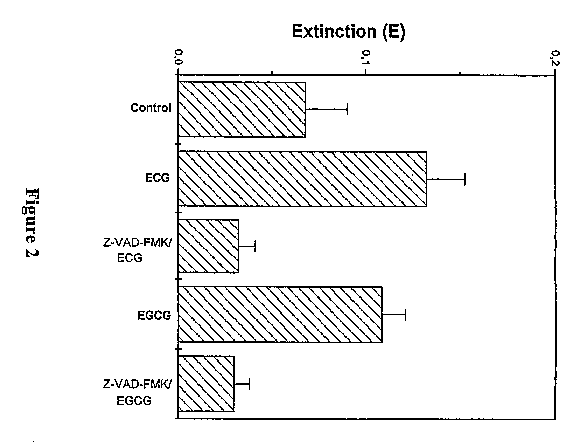

Moreover, catechins at concentrations of 80 µg/ml induce apoptosis causing a significant increase in the caspase 3 protease activity compared with untreated cells. Figure 2 shows the activation of the protease activity exerted by ECG and EGCG alone and in combination with a caspase 3 specific inhibitor (Z-VAD-FMK) expressed as arbitrary units referred to the optical density of the used instrumentation.

The invention is illustrated in greater detail by the following examples.

### EXAMPLE 1

### Preparation of standards and samples

Standards consisted of a mixture of (+)-catechin (C), (-)-epicatechin (EC), (-)-epigallocatechin-3-gallate (EGCG), (-)-epicatechin-3-gallate (ECG), gallic acid (GAc), theophylline (T), caffeine (Ca), chlorogenic acid (CIAc), caffeic acid (Cac), catechol (Ct) and tea extracts, containing teaflavin, teaflavin-3-monogallate, teaflavin-3'-monogallate and teaflavin-3,3'-digallate (with purity >80%) were prepared dissolving known amounts of all the analytes in the mobile phase used for the chromatographic separation so as to reach concentrations from 0.3 to 500 µg/ml. Samples were prepared by infusing 1 g of carob leaves or pod meal in 100 ml of hot distilled water for 15 min. Afterwards, the aqueous extract was centrifuged at 13,000 rpm for 10 min, then filtered through PTFE 0.22 µm filters. A second aqueous extract was obtained from the centrifugation residue.

### EXAMPLE 2

### Chromatographic analysis

The chromatographic separation was carried out with an analytic Microsorb RP-C₁₈ 100 A column (250 X 4.6 mm), thermostatized at 40°C, using a gradient with starting composition: 68% water, 22% of 0.5% phosphoric acid, 4% acetonitrile and 6% methanol, linearly increasing the organic solvents with a flow of 1 ml/min. UV-Vis absorption spectra (λ= 198-708 nm) were recorded continuously and the samples absorption peaks were identified by comparing retention times and UV-Vis spectra with those of the standard. The peak area at 205 nm was used to establish calibration curves.

**TABLE 1. Catechins quantitation by chromatographic analysis**

| | CAROB LEAVES | | | CAROB MEAL | | |
|---|---|---|---|---|---|---|
| | mg/g ± s.d.* | | | mg/g ± s.d.* | | |
| | 1^{st} infusion | 2^{nd} infusion | Total | 1^{st} infusion | 2^{nd} infusion | Total |
| G Ac | 3.34 ± 0.032 | 0.96 ± 0.020 | 4.30 | 0.87 ± 0.000 | 0.33 ± 0.007 | 1.20 |
| EGC | - | - | - | 0.05 ± 0.000 | 0.01 ± 0.001 | 0.06 |
| C | - | - | - | 0.01 ± 0.000 | - | 0.01 |
| EGCG | 1.23 ± 0.409 | 0.28 ± 0.030 | 1.51 | 0.01 ± 0.000 | - | 0.01 |
| EC | - | - | - | - | - | - |
| ECG | 0.47 ± 0.095 | - | 0.47 | 0.06 ± 0.000 | 0.02 ± 0.004 | 0.08 |
| Total | 5.04 ± 0.395 | 1.24 ± 0.049 | 6.28 | 1.00 ± 0.008 | 0.36 ± 0.002 | 1.36 |

| | | | | | | |
|---|---|---|---|---|---|---|
| * = Means and Standard Deviations for 3 determinations | | | | | | |

### EXAMPLE 3

### Pharmacological experimentation

### Cell cultures

Tumour cell cultures (T1) used in the tests derive from transgenic mice hepatocellular carcinoma, in which an over-expression of the *c*-myc and TGFα had been induced. Cells were kept in L-glutamine-free DMEM medium, HAM F 12 containing 1 mg/ml galactose, 18 mM HEPES, 5 mM Na piruvate, 30 mg/Ml proline, ITS 100 X, 2 mM glutamine, 0.1% gentamicin (Sigma-Aldrich, Italy) and added with 10% FBS. Cell lines were cultured in polystyrene plates (Dasit, Italy).

### Cell proliferation assay

T1 cells were plated in 96 wells-plates at a concentration of 10,000 cells/well and left to sediment for 4-5 hours. Cells were then incubated in EGCG or ECG at a concentration of 20, 40, 60, 80, 160 µg/ml for 24 and 48 hours. The substances were diluted in the culture medium starting from stock solutions (aqueous solution for EGCG and aqueous-ethanol solution (1:1) for ECG). Control cells were treated with solutions containing ethanol in amounts corresponding to that of the samples containing catechins. After 24 or 48 hours, the proliferation was colorimetrically measured using a specific kit (Cell Titer Aqueous Cell Proliferation Assay, Promega) which makes use of a colorimetric transformation reaction of colourless MTS (tetrazolium salt) into coloured formazan by living, proliferating cells. Formazan quantitation was carried out by spectrophotometric analysis at λ = 490 nm.

### Evaluation of caspase 3 activation

Caspase 3 protease activity in cells lysates was determined using a Promega kit. T1 Cells were treated with EGCG and ECG (80 µg/ml) alone or in combination with a caspase inhibitor (Z-VAD-FMK) for 24 or 48 hours. Afterwards a colorimetric substrate (DEVD-pNA) able to evidence the protease activity was added. After incubation for 4 hours at 37°C, the protease activity was determined by spectrophotometric analysis at λ = 405 nm.

## Claims

1. The use of an aqueous extract of *Ceratonia siliqua* leaves and pod containing flavonoids for the preparation of medicaments, dietary supplements or alimentary products with antioxidant and antitumor activities.

2. The use as claimed in claim 1, wherein the *Ceratonia siliqua* extract is a dry extract.

3. The use of an extract as claimed in claims 1-2, wherein flavonoids are gallic acid, (-)-epigallocatechin gallate, (-)-epicatechin gallate.

4. A process for the preparation of *Ceratonia siliqua* leaves and pod extracts containing flavonoids comprising the following steps:
a) infusing the leaves or pod meal in hot distilled water;
b) centrifuging the aqueous extract;
c) filtering the aqueous extract;
d) evaporating off water;
e) optionally repeating steps a)-d) on the centrifugation residue from step b),

5. A process as claimed in claim 4, wherein 50 to 200 ml of water are used per 1 g of leaves or pod meal.

6. A process as claimed in claim 4, wherein 100 ml of water are used per 1 g of leaves or pod meal.

7. Extracts obtainable by the process of claims 4 - 6.

8. Pharmaceutical preparations, dietary supplements or alimentary products containing the extract of claim 7.

## Patentansprüche

1. Die Verwendung eines wässrigen Extraktes von Blättern und Hülsen von *Ceratonia siliqua,* enthaltend Flavonoide, für die Herstellung von Medikamenten, Nahrungsergänzungsmitteln und Nahrungsmittelprodukten mit Antioxidations- und Antitumorwirkungen.

2. Die Verwendung wie in Anspruch 1 beansprucht, worin der Extrakt von *Ceratonia siliqua* ein Trockenextrakt ist.

3. Die Verwendung eines Extraktes wie in den Ansprüchen 1 oder 2 beansprucht, worin die Flavonoide Gallussäure, (-)-Epigallocatechingallat, (-)-Epicatechingallat sind.

4. Ein Verfahren für die Herstellung von Extrakten aus Blätter und Hülsen von *Ceratonia siliqua,* enthaltend Flavonoide, umfassend die folgenden Schritte:
a) Durchziehenlassen von Blättern oder Hülsenmehl mit heißem destillierten Wasser;
b) Zentrifugieren des wässrigen Extraktes;
c) Filtern des wässrigen Extraktes;
d) Abdampfen des Wassers;
e) gegebenenfalls Wiederholen der Schritte a) bis d) an dem Zentrifugationsrückstand aus Schritt b).

5. Ein Verfahren wie in Anspruch 4 beansprucht, worin 50 bis 200 ml an Wasser pro 1 g an Blättern oder Hülsenmehl verwendet werden.

6. Ein Verfahren wie in Anspruch 4 beansprucht, worin 100 ml an Wasser pro 1 g an Blättern oder Hülsenmehl verwendet werden,

7. Extrakte, erhälfilich durch das Verfahren gemäß Ansprüche 4 bis 6.

8. Pharmazeutische Präparationen, Nahrungsergänzungsmittel und Nahrungsmittelprodukte, enthaltend den Extrakt nach Anspruch 7.

## Revendications

1. Utilisation d'un extrait aqueux de feuilles et de gousses de *Ceratonia siliqua* contenant des flavonoïdes pour la préparation de médicaments, de suppléments diététiques ou de produits alimentaires ayant une activité antioxydante et antitumorale.

2. Utilisation selon la revendication 1, dans laquelle l'extrait de *Ceratonia siliqua* est un extrait sec.

3. Utilisation d'un extrait selon les revendications 1 et 2, dans laquelle les flavonoïdes sont l'acide gallique, le gallate de (-)-épigallocatéchine, le gallate de (-)-épicatéchine.

4. Procédé pour la préparation d'extraits de feuilles et de gousses de *Ceratonia siliqua* contenant des flavonoïdes comprenant les étapes suivantes :
a) infuser les feuilles ou la farine de gousses dans de l'eau distillée chaude ;
b) centrifuger l'extrait aqueux ;
c) filtrer l'extrait aqueux ;
d) évaporer l'eau ;
e) éventuellement, répéter les étapes a) à d) sur le résidu de centrifugation de l'étape b).

5. Procédé selon la revendication 4, dans lequel 50 à 200 ml d'eau sont utilisés pour 1 g de feuilles ou de farine de gousses.

6. Procédé selon la revendication 4, dans lequel 100 ml d'eau sont utilisés pour 1 g de feuilles ou de farine de gousses.

7. Extraits pouvant être obtenus par le procédé des revendications 4 à 6.

8. Préparations pharmaceutiques, suppléments diététiques ou produits alimentaires contenant l'extrait de la revendication 7.
